# EUROPEAN PATENT APPLICATION

(11) **EP 1 897 551 A1**
(43) Date of publication of application: **12.03.2008**
(21) Application number: 06746005.5
(22) Date of filing: 02.05.2006
(51) Int. Cl.: A61K 38/17, A61K 31/352, A61K 31/353, A61K 31/7008, A61K 31/7048, A61P 19/02, A61P 19/08, A61P 29/00, A23L 1/30, A23L 1/305, A23L 1/333

(54) **HEALTH FOOD AND PHARMACEUTICAL COMPOSITION FOR AMELIORATION OF DISEASE INDUCED BY METABOLIC DISORDER IN CARTILAGE**

(30) Priority: 06.05.2005 JP 2005135120
(71) Applicant: AMINO UP CHEMICAL CO. LTD., Sapporo-shi, Hokkaido 004-0839 (JP); Ishida Pharma Corporation, Tsu-shi Mie, 5140838 (JP)
(72) Inventor: UCHIDA, Atsumasa, 5140008 (JP); WAKAME, Koji c/o Amino Up Chemical Co., Ltd., Sapporo-shi, Hokkaido 0040839 (JP); ISHIDA, Tsuyoshi c/o Ishida Pharma Corporation, Mie 5140838 (JP)
(74) Representative: Horner, Martin Grenville
(86) International application number: PCT/JP2006/309155
(87) International publication number: WO 2006/120974

(57) **Abstract**

The invention relates to a health food and a pharmaceutical preparation, containing a composition for treating rheumatoid arthritis. The health food and pharmaceutical preparation in which glucosamine, a collagen and an isoflavone aglycone act synergically, which are useful for relieving symptoms or treatment of diseases related to abnormal cartilage metabolism, especially rheumatoid arthritis.

## Description

### TECHINICAL FIELD

The present invention relates to a health food and a pharmaceutical preparation containing a composition in which a glucosamine having anti-inflammatory effect, a collagen having oral tolerance effect and an isoflavone aglycone having antiangiogenic property act synergically, which are useful for relieving symptoms or treatment of diseases related to abnormal cartilage metabolism, especially rheumatoid arthritis.

### BACKGROUND ART

As the society ages, the number of patients with chronic diseases called lifestyle-related diseases caused by aging is rapidly increasing. In declines in physical function and kinesthetic function of elderly people, diseases induced by abnormal cartilage/bone metabolism, such as knee osteoarthritis, osteoarthritis of the spine and osteoporosis, which are related to hard tissues such as bone and peripheral tissues. Particularly, theses diseases are accompanied by strong pain in many cases, deteriorating quality of life. Currently, the numbers of patients with these diseases are estimated as about 7 million for knee osteoarthritis, about 15 million for osteoarthritis of the spine and about 7 million for osteoporosis.

As treatments for these diseases, drug treatment using nonsteroidal anti-inflammatory drugs (NSAIDs), steroidal anti-inflammatory drugs or immune-regulating drug, physical therapy such as rehabilitation, and treatment by surgery such as artificial joint replacement have been employed.

These conventional treatment methods, which cannot achieve satisfactory treatment effects, generally produce strong side effects such as organ derangement and hypersensitivity or such drug treatment may induce other diseases (such as steroid osteoporosis and interstitial pneumonia), thereby inflicting suffering on patients. Since the pathogenesis is unclear, there has lacked any fundamental treatment available. It is often the case that patients put up with pains at home by only applying a simple treatment like poultice when their symptoms are relatively mild or with no appropriate treatment being available.

Under these circumstances, treatment method conveniently and continuously usable for a long period of time both at home and on the medical wards and free of side effects has been demanded for critical prevention of these diseases, prevention of disease progresses and treatment of the diseases.

Glucosamine is a decomposition product of chitin shells of crabs and shrimps generally contain in a large amount and is a kind of hexosamine produced by removing acetyl group from monosaccharide, N-acetylglucosamine. Glucosamine is a typical natural amino sugar, which constitutes proteoglycan forming connective tissues and cartilages and contributes to enhancing strength, flexibility or resilience of the site in each tissue in combination with collagen fibers and water content. Glucosamine, not only constitutes cartilages or connective tissues but also reportedly has anti-inflammatory action via neutrophil to be effective for treatment of arthritis (Japanese Patent Application Laid-Open No.2004-52860; Patent Document 1 (Relevant Application; WO02/043653 pamphlet)).

Collagen, inherently existing in the living body, is a fibrous protein which plays a role in connecting cells with cells. Especially, it is contained in large amount in hard tissues such as skin, cartilages and tendons, cartilage tissues and connective tissues. In human body, collagen accounts for about 30 % of all proteins and if water content is excluded, collagen, which constitutes 70 % of the skin, 60 % of a tooth root and 50 % of a cartilage, is one of proteins most important for the body structure. In arthrodial cartilage, II-type collagen is the principal component and is thought to be the source of immunity in rheumatoid arthritis. For patients with rheumatoid arthritis, oral tolerance therapy using immune tolerance mechanism by oral intake of II-type collagen is being developed.

Isoflavone is a kind of polyphenol, which is abundantly contained in legume plants. Since its structure is similar to that of female hormone, it is called plant estrogen. Isoflavone is contained in large amount in soybean where most of isoflavone is present as glycoside while in soybean fermented foods, isoflavone, fermented by microorganisms, is present as aglycone.
The present inventors developed a composition (Genistein Combined Polysaccharide:GCP) having antitumor action and antiangiogenic action synergistically enhanced by fermenting isoflavone derived from legume plants with a fungus *basidiomycete* to convert the isoflavone into aglycone and allow it to contain *basidiomycete* polysaccharide having antitumor effect and immunostimulatory action. (WO01/44488 pamphlet: Patent Document 2(Relevant Application; US Patent Application Publication No. 2003/068329 specification)).

Recently, it has been revealed that in articular inflammations including rheumatoid arthritis, neoangiogenesis plays a significant role by contributing to progress or persistence of diseases through supply of nutrition or oxygen to inflammatory cells. There has been a suggestion that antiangiogenic action is useful in process of treating articular inflammations including rheumatoid arthritis (J. Cell. Mol. Med. vol.6, No.3, 357-376, 2002:Non-Patent Document 1, Arthritis Res. 4(Suppl. 3) S81-S90, 2002: Non-Patent Document 2). For rheumatoid arthritis, there have been attempt made to make clinical application of shark cartilage extract said to have antiangiogenic action, however, since shark cartilage extract is disadvantageous in that it has characteristic ammonia odor and that in order to intake an effective amount, it must be ingested in so large an amount as several tens of grams that it is difficult to continuously ingest such an amount for a long period of time, it has not been out into practical use.

[Patent Document 1] Japanese Patent Application Laid-Open No. 2004-529860
[Patent Document 2] International Patent Publication No. WO01/44488 pamphlet
[Non-Patent Document 1] J. Cell. Mol. Med. vol.6, No.3, 357-376, 2002
[Non-Patent Document 2] Arthritis Res. 4(Suppl. 3) S81-S90, 2002

### DISCLOSURE OF INVENTION

### PROBLEM TO BE SOLVED BY INVENTION

The object of the present invention is to a health food and a pharmaceutical preparation containing a composition in which substances having anti-inflammatory effect, oral immune tolerance effect and antiangiogenic property act synergically to relieve symptoms or help treat rheumatoid arthritis.

### MEANS FOR SOLVING THE PROBLEM

As a result of intensive studies with a view to solving the above problem, the present inventors have found out that by ingesting glucosamine having anti-inflammatory action, collagen promoting oral tolerance mechanism and isoflavone aglycone having antiangiogenic effect at the same time, symptoms such as pains and inflammations in rheumatoid arthritis can be alleviated and recurrence of pains after treatment can be prevented with synergistic action of the substances, thereby completed the invention.

That is, the present invention relates to a health food and a pharmaceutical preparation containing a substance which contains glucosamine, collagen, isoflavone aglycone to act synergically to relieve symptoms or help treat rheumatoid arthritis.
1. A composition containing glucosamine, collagen and isoflavone aglycone.
2. The composition according to 1, wherein the glucosamine is obtained as acid hydrolysate of structural polysaccharide of chitin contained in arthropods and/or mollusks.
3. The composition according to 2, wherein the glucosamine is hydrochloride salt of glucosamine.
4. The composition according to 1, wherein the isoflavone aglycone is genistein.
5. A composition comprising 30 to 90 mass % of hydrochloride salt of glucosamine, 1 to 10 mass % of II-type collagen as peptide, and 3 to 30 mass % of isoflavone aglycone (provided that the total amount of these three components is 100 %).
6. The composition according to any one of 1 to 5, which is a composition for a health food for treating, prevention and/or alleviating a disease related to abnormal cartilage metabolism.
7. The health food according to 6, wherein the disease related to abnormal cartilage metabolism is rheumatoid arthritis, knee osteoarthritis or osteoarthritis of the spine.
8. The composition according to any one of 1 to 5, which is a medical preparation for treating, prevention and/or alleviating a disease related to abnormal cartilage metabolism.
9. The composition for medical preparation according to 8, wherein the disease related to abnormal cartilage metabolism is rheumatoid arthritis, knee osteoarthritis or osteoarthritis of the spine.

### BEST MODE FOR CARRYING OUT THE INVENTION

Hereinafter, the present invention is described in detail.
The composition containing glucosamine having anti-inflammatory action, collagen promoting oral tolerance mechanism and isoflavone aglycone having antiangiogenic effect enables simultaneous intake of these components so that the components can act synergically to alleviate symptoms such as pains and inflammation in rheumatoid arthritis and prevent recurrence of pain after treatment.

Glucosamine used in the present invention can be obtained as acid hydrolysate of structural polysaccharide chitin contained in arthropods such as crustacean and coleopteran and/or mollusks such as cephalopoda and gastropoda or as crude purification product containing glucosamines obtained in decomposition process or as a component isolated therefrom.

The form of the above glucosamine is not particularly limited, however, preferred examples include glucosamine and its hydrochloride salts or sulfate salts and N-acetyl glucosamine. Most preferred is hydrochloride salt of glucosamine.

There is no particular limitation on the origin of collagen used in the present invention and examples of collagen usable in the present invention include extracts containing collagen from hard tissues, cartilage tissues, skin tissues and organs of mammals such as cow, pig, sheep, goat, horse and rabbit, avian species such as chicken, turkey, ostrich and emu, fishes such as tuna, bonito, eel, salmon, shark and ray, reptiles such as turtle and snake, mollusks such as squid and octopus and arthropods. Also, peptides obtainable by subjecting such an extract to modification, hydrolysis and enzymatic decomposition according to conventional method or collagens chemically synthesized based on the amino-acid sequence or gene arrangement of each of the above animals can be used.

The collagen, which can be a source for oral tolerance in rheumatoid arthritis, is II-type collagen. Therefore, preferred are those obtained by isolating only II-type collagen from an extract containing collagen and then purifying it.

Examples of isoflavone aglycone used in the present invention include those of legume plants such as soybean, red clover, white clover, kuzu, alfalfa, *Lathyrus palustris,* Psoralea corylifolia, and *Cytisus scoparius* and extracts obtained by extracting tissues of these plants with organic solvent such as water and alcohol or a mixture thereof. Also, those obtained by fermenting the material with β-glicosidase produced by a plant or microorganism to convert isoflavone glycosides into aglycones can be used. Miso(soybean paste), soy sauce and natto which are fermented food of soybeans can be also employed as isoflavone-containing materials as long as they contain isoflavone.

Particularly, by fermenting isoflavone glycosides in legume plants in mixed culture with *Basidiomycetes* to convert the isoflavones into aglycones and allow the composition to contain *Basidiomycetes* polysaccharides having anti-tumor and immunostimulatory actions, the composition (GCP: registered trademark of Amino Up Chemical Co., Ltd.) can have anti-tumor and immunostimulatory actions synergistically enhanced and can be used most suitably in the present invention.

The composition in the present invention contains glucosamine having anti-inflammatory action, collagen promoting oral tolerance mechanism and isoflavone aglycone having antiangiogenic effect. The composition can be used in orally-ingested forms, so that by ingesting these components at the same time, the components act synergistically to alleviate symptoms such as pains and inflammations in rheumatoid arthritis or to prevent recurrence of pains after treatment. The ratio of glucosamine to collagen to isoflavone aglycone in the composition of the present invention can be selected within an extent that the effects of the present invention can be obtained. One example of the ratio is from 30 to 90 mass % of glucosamine hydrochloride, from 1 to 10 mass % of II-type collagen as peptide and from 3 to 30 mass % of isoflavone aglycone, based on the total amount of the three components as 100 %. Preferred is from 30 to 45 mass % of glucosamine hydrochloride, from 1.5 to 5 mass % of II-type collagen as peptide and from 3 to 15 mass % of isoflavone aglycone.
If the amount of each component is less than the lower limit or exceeds the upper limit, glucosamine, collagen and isoflavone aglycone cannot act synergistically.
In a case where the composition of the present invention is to be orally ingested, the composition can be used in form of tablet, pill, capsule, power, granule or syrup. When the composition is processed into such a form, appropriate additives (excipient, nutritive component, extender, sweetening agent, spicery, colorant, antiseptic agent and emulsifier) may be added within an extent that the effects of the invention are not impaired.

In a case where the composition of the present invention is orally ingested, the ingestion dose depends on the sex, age, body weight, symptoms and target effects of treatment. Generally, the dose may be ingested such that from 100 to 2000 mg of glucosamine, from 10 to 1000 mg of collagen and from 5 to 500 mg of isoflavone aglycone (each in terms of dry substance) can be taken per adult per day. Preferably, the composition is ingested such that from 500 to 1000 mg of glucosamine, from 20 to 40 mg of collagen and from 50 to 100 mg of isoflavone aglycone (each in terms of dry substance) can be taken per adult per day.

### EXAMPLES

The present invention is specifically described by referring to Examples and Test Examples below. However, the present invention is by no means limited to the Examples.

### Example 1: Production example of capsules

Each component described in the formulation below was well stirred and mixed together according to a conventional manner (Sample 1).

### [Table 1]

**Table 1**

| Components | Blended amount (mass %) |
|---|---|
| Glucosamine hydrochloride derived from shrimps and crabs (product of Koyo Chemical Company Limited; containing Glu cosamine hydrochloride at 98 % or more) | 39.3 |
| II-type-collagen-containing extract derived from chicken cart ilage (product of Nippon Meat Packers, Inc.; containing collagen pepti de at 30 % or more) | 7.9 |
| Basiodiomycetes-fermentation product (GCP) of Isoflavone-rich so ybean extract (product of Amino Up Chemical Co., Ltd.; containing isoflavone aglycone at 15.69%) | 39.3 |
| Ascorbic acid (product of Tanabe Seiyaku Co., Ltd.) | 13.5 |
| Total | 100.0 |

### Test Example 1: Test for effects on rheumatoid arthritis model animal

To MRL/lpr mice which naturally develop systemic lupus erythematosus as autoimmune abnormality to cause multiple arthritis and enlarged lymph nodes and are generally used as rheumatoid arthritis model animal, feed was provided in each test section as follows.
Eight of the mice were used per section and bred for 72 days with free access to feed and water.
Test Group 1: Normal feed (CE-2 mouse feed produced by CLEA Japan, Inc.),
Test Group 2: Normal feed into which GCP to be ingested per 1000 mg/kg body weight per day was blended.
Test Group 3: Normal feed into which Sample 1 to be ingested per 630 mg/kg body weight per day was blended.

### Results

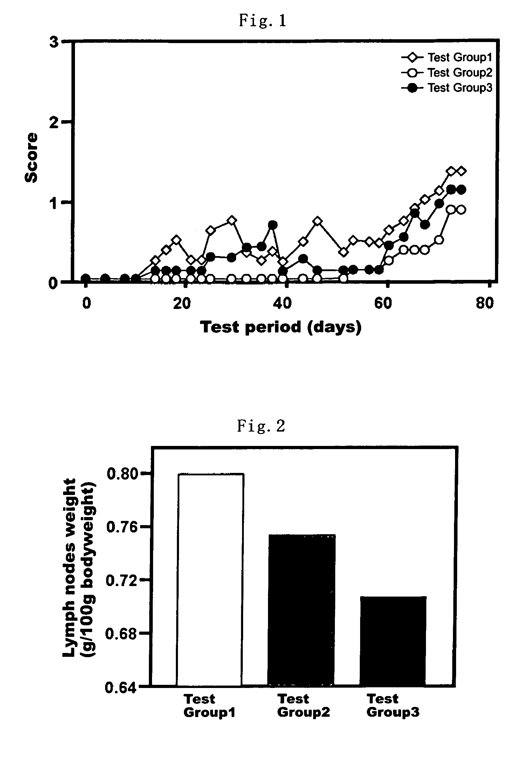

The conditions of rheumatoid arthritis during the testing period were rated on a scale of one to four and indicated by scores. (The more rheumatoid arthritis advanced, the larger the score.) The changes are shown in Fig. 1. In Test Example 1, as the testing period continued, the condition of rheumatoid arthritis worsened to increase the score. In the other hand, in Test Examples 2 and 3, increase in the scores was repressed.

Values of mass of lymph nodes per body weight at the time of completion of the Tests are shown in Fig.2. In Test Example 1, enlarged lymph nodes were observed and the mass was increased while in Test Examples 2 and 3, enlargement of lymph nodes was suppressed and especially in Test Example 3, the suppression effect was significant.

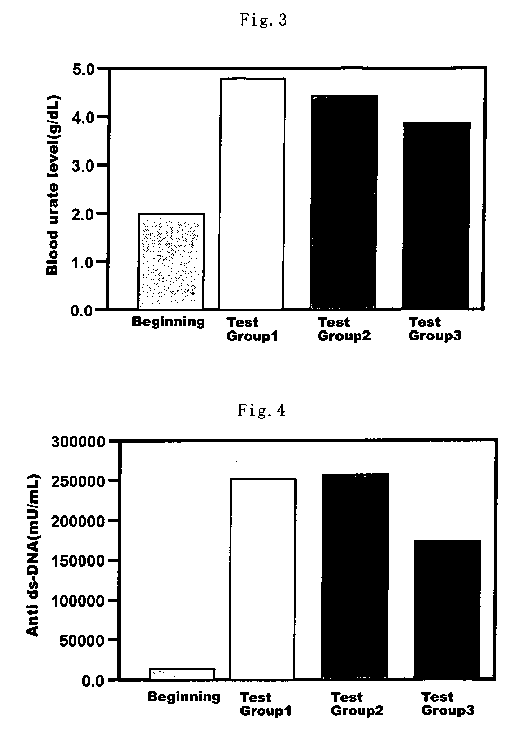

As rheumatoid arthritis advances, blood urate level increases. Blood urate levels at the time of completion of the Tests were measured by using a commercially available kit for biochemically analyzing blood serum (product of Wako Pure Chemical Industries, Ltd.; Urate C Test Wako). The results are shown in Fig. 3. The value was highest in Test Example 1 while in Test Examples 2 and 3, the values were low as compared with Test Example 1 and in Test Example 3, the value was lowest.

The amount of anti-double-stranded DNA (ds-DNA) antibody in the blood as biomarker which increases in rheumatoid arthritis was measured by using an ELISA kit (product of Shibayagi Co., Ltd., Lbis anti-ds-DNA mouse). The results were shown in Fig. 4. In Test Example 1, the amount of anti-ds DNA antibody increased as compared with the amount before administration, and the results were similar in Test Example 2. In Test Example 3, increase in the amount of anti-ds DNA antibody was suppressed, which suggests that development in disease state of rheumatoid arthritis was suppressed.

It has been revealed from the above results that in MRL/lpr mice used as animal model for rheumatoid arthritis, the composition of the present invention can suppress swelling of lymph nodes, to thereby suppress development of rheumatoid arthritis.

### Test Example 2:Clinical studies on patients with rheumatoid arthritis

Double blind clinical tests were conducted on 87 patients with rheumatoid arthritis to verify effectiveness of the composition of the present invention. Pullulan-made hard capsule were filled up with Sample 1 and thus hard-capsule preparations each containing 0.32 g of Sample 1 were prepared (positive control drug group). As placebo, powder prepared by adding dextrin to malt extract to make its appearance indistinguishable from the positive control drug was used in form of hard capsule (placebo group).

After obtaining informed consents, the patients were administered 12 capsules of the positive control drug or the placebo drug in total (divided into 3 doses) per day. The administration was conducted for 12 weeks on patient groups selected in a random manner as shown in the Table 2.

### [Table 2]

**Table 2**

| | Male | Female | Age |
|---|---|---|---|
| Positive Control 43 patients | 6 patients | 37 patients | 59.1 on an average (30-70) |
| Placebo 44 patients | 5 patients | 39 patients | 57.1 on an average (29-74) |

Evaluated items include the number of joints with pain, the number of swelled joints and the CRP value in the blood, and also include the degree of arthritic pain as subjective symptom and the degree of life activities, which were evaluated by patients themselves on a zero-to-10 scale (zero : free from pain and feeling no difficulty in life activities, 10: Very painful and totally unable to do life activities) by selecting from numbers representing conditions, one they judged subjectively as most suitable for explaining their own conditions in questionnaires conducted before administration and 3 months after the administration was started, and the results were compared and studied.

### Results

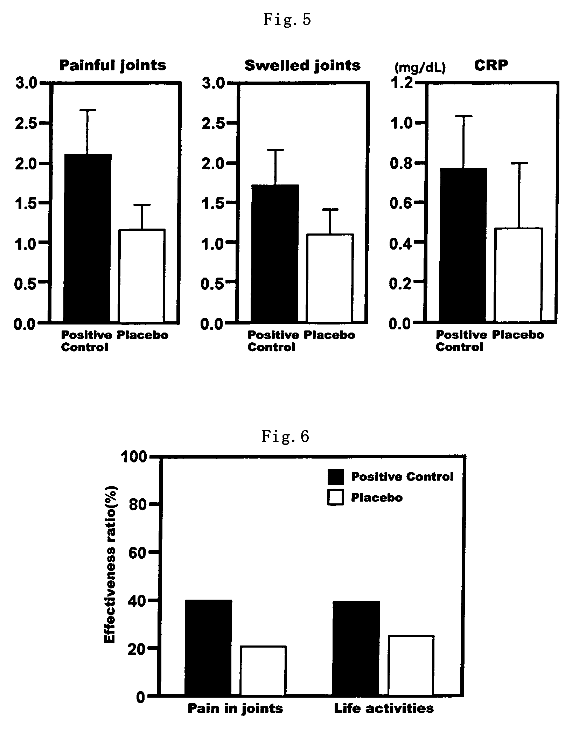

Improvement degrees in the number of joints with pain, the number of swelled joints and the C-reactive protein (CRP) value in the blood in comparison between the positive control drug group and the placebo group before and after administration are shown in Fig. 5. In all of the items, great improvements were observed in the positive control drug group as compared with the placebo group, which suggests that intake of the substance of the present invention can alleviate pain and swelling in joints caused by rheumatoid arthritis and improve inflammatory conditions.

In the zero-to-10 scale evaluations made by the patients on pains in joints as subjective symptom and on life activities, effectiveness ratio was calculated by judging an item where the evaluation after administration was improved from before-administration by one or more grades as effective, and the results are shown in Fig. 6. In all the items, the effectiveness ratio was close to 40 % in the positive control group while 20 or 25 % in the placebo group. The positive control group was evaluated as more effective than the placebo group in all evaluation items.

### Test Example 3 : Clinical studies on a patient with rheumatoid arthritis

Pullulan-made hard capsule were filled up with Sample 1 and thus hard-capsule preparations each containing 0.32 g of Sample 1 were prepared. The drug was administered in 9 capsules a day (divided into 3 doses) to a subject person (female aged 55) having too a bad liver to take strong medications, for 4 weeks.
The evaluation items and method are the same as in Test Example 2, and evaluations were made before administration and 1 month after administration was started and the results were compared and are shown in Table 3.

### [Table 3]

**Table 3**

| | Before administration | 1 month after administration was started |
|---|---|---|
| Number of painful joints | 4.0 | 2.0 |
| Number of swelled joints | 6.0 | 3.0 |
| CRP value | 8.3 | 3.1 |

### Results

The subject person underwent an operation on the left knee for artificial joint replacement during the administration period. The number of painful joints decreased 1 month after administration was started as compared with that of before-administration, with improvement in each of the values. It suggests that intake of the substance of the present invention alleviated the pain and swelling of joints caused by rheumatoid arthritis and improved the inflammatory condition.

### Test Example 4 : Clinical studies on a patient with rheumatoid arthritis

The hard-capsule preparations containing Sample 1 was administered in the same manner as in Test Example 3 to a subject person (female aged 51), in combination with conventional treatment for rheumatoid arthritis she had received but by which had gained little improvement. The evaluations were made before administration, and 2 months, 3 months and 4 months after the administration was started for comparison and the results are shown in Table 4.

### [Table 4]

**Table 4**

| | Before administration | 2 months after administration was started | 3 months after administration started was | 4 months after administration started |
|---|---|---|---|---|
| Number of painful joints | 13.0 | 13.0 | 13.0 | 8.0 |
| Number of swelled joints | 7.0 | 10.0 | 10.0 | 6.0 |
| CRP value | 8.6 | 3.86 | | 3.83 |

### Results

Improvement was observed in the CRP values, which suggests that intake of the substance of the present invention improved the inflammatory condition.

### Test Example 5: Clinical studies on a patient with rheumatoid arthritis

The hard-capsule preparations containing Sample 1 was administered in the same manner as in Test Example 3 to a subject person (female aged 60), in combination with conventional treatment for rheumatoid arthritis she had received but by which had gained little improvement. The evaluations were made before administration, and 2 months, 3 months and 4 months after the administration was started for comparison and the results are shown in Table 4.

### [Table 5]

**Table 5**

| | Before administration | 1 month after administration was started | 2 months after administration was started | 3 months after administration was started | 4 months after administration was started |
|---|---|---|---|---|---|
| Number of painful joints | 29.0 | | 13.0 | 13.0 | 8.0 |
| Number of swelled joints | 32.0 | | 10.0 | 10.0 | 6.0 |
| CRP value | 3.5 | 4.06 | 3.06 | | 2.12 |

### Results

Improvement was observed in the CRP values, which suggests that intake of the substance of the present invention improved the inflammatory condition.

### BRIEF DESCRIPTION IF DRAWINGS

[Fig. 1] Changes in scores in rheumatoid arthritis model animals (MRL/lpr mice) observed during the test period.
[Fig. 2] The weights of lymph nodes per body weight at the time when the tests on rheumatoid arthritis model animals were completed.
[Fig. 3] The blood urate levels at the time when the tests on rheumatoid arthritis model animals were completed.
[Fig. 4] The amounts of anti-double-stranded DNA (ds-DNA) antibody in the blood at the time when the tests on rheumatoid arthritis model animals were completed.
[Fig. 5] Comparison between numbers of painful joints and swelled joints and CRP values.
[Fig. 6] Effectiveness ratios with respect to improvements of subjective symptoms, based on self-evaluation of patients on pains in joints and conditions of life activities.

## Claims

1. A composition containing glucosamine, collagen and isoflavone aglycone.

2. The composition according to claim 1, wherein the glucosamine is obtained as acid hydrolysate of structural polysaccharide of chitin contained in arthropods and/or mollusks.

3. The composition according to claim 2, wherein the glucosamine is hydrochloride salt of glucosamine.

4. The composition according to claim 1, wherein the isoflavone aglycone is genistein.

5. A composition comprising 30 to 90 mass % of hydrochloride salt of glucosamine, 1 to 10 mass % of II-type collagen as peptide, and 3 to 30 mass % of isoflavone aglycone (provided that the total amount of these three components is 100 %).

6. The composition according to any one of claims 1 to 5, which is a composition for a health food for treating, prevention and/or alleviating a disease related to abnormal cartilage metabolism.

7. The health food according to claim 6, wherein the disease related to abnormal cartilage metabolism is rheumatoid arthritis, knee osteoarthritis or osteoarthritis of the spine.

8. The composition according to any one of claims 1 to 5, which is a medical preparation for treating, prevention and/or alleviating a disease related to abnormal cartilage metabolism.

9. The composition for medical preparation according to claim 8, wherein the disease related to abnormal cartilage metabolism is rheumatoid arthritis, knee osteoarthritis or osteoarthritis of the spine.
